# EUROPEAN PATENT SPECIFICATION

(11) **EP 2 075 030 B1**
(45) Date of publication and mention of the grant of the patent: **02.06.2010**
(21) Application number: 08172416.3
(22) Date of filing: 19.12.2008
(51) Int. Cl.: A61M 25/098

(54) **Guide wire**
Führungsdraht
Guide-fil

(30) Priority: 28.12.2007 JP 2007340858; 25.01.2008 JP 2008015575
(43) Date of publication of application: 01.07.2009
(60) Divisional application: 09179184.8
(73) Proprietor: Terumo Kabushiki Kaisha, Shibuya-ku Tokyo (JP)
(72) Inventor: Kinoshita, Yasushi, Shizuoka (JP); Kobayashi, Junichi, Shizuoka (JP); Kousai, Tadashi, Shizuoka (JP)
(74) Representative: Dossmann, Gérard

(56) References cited:
- EP-B- 0 759 793
- US-A- 5 606 981

## Description

The present invention relates to a guide wire.

A guide wire is used to facilitate insertion of a catheter into the lumen (such as digestive tract and blood vessel) of a living body. When in use, a catheter is slipped on it.

A guide wire is also used to lead an endoscope or a catheter inserted into the lumen of an endoscope to a desired position in the lumen of a living body at the time of observation or treatment of the lumen of a living body (See Japanese Patent Laid-Open No. 2007-135645).

EP-B-0759793 discloses a guide wire comprising a long wire proper and a radiopaque part. The radiopaque part has a radiopaque member in form of a metallic coil and a coating loaded with radiopaque material. The coating comprises sections of different radiopacity.

A guide wire for this purpose consists of a long wire proper and a coil covering the distal end of the wire proper. The coil may be formed from a radiopaque material such as noble metal. The coil makes the distal end of the guide wire visible to facilitate insertion of the guide wire into a living body by radioscopy. The coil consists of two parts (the first coil and the second coil), and each part is formed from a helically wound wire.

The conventional coil consisting of two parts has the disadvantage of being recognized as one monochromatic continuous member in radioscopy with a certain intensity because the two parts are close to each other. This disadvantage causes difficulties in locating the distal end of the guide wire being inserted into the lumen of a living body.

Moreover, the conventional coil alone does not produce a high-contrast X-ray image. This causes difficulties in locating the distal end of the guide wire and hence hinders smooth insertion of the guide wire into a living body- The fact that the guide wire is monochromatic and merely provides a low-contrast X-ray image makes it difficult to determine how far the guide wire has advanced and to locate the distal end of the guide wire being inserted into a living body.

It is an object of the present invention to provide a guide wire which produces a high-contrast X-ray image and facilitates its location at the time of insertion into the lumen of a living body.

The foregoing object is achieved by the present invention defined in the appended claims.

It is desirable that the radiopaque member in the first and third radiopaque regions should be arranged 3 to 7 times as densely as the radiopaque member in the second radiopaque region.

It is desirable that the content of the radiopaque material in the resin coating layer in the second radiopaque region should be less than 10% of the content of the radiopaque material in the resin coating layer in the first radiopaque region.

It is desirable that the first radiopaque region and the third radiopaque region should be similar to each other in ability to form an X-ray image.

It is desirable that the first radiopaque region should be longer than the second radiopaque region, and the second radiopaque region should be equal to or longer than the third radiopaque region.

It is desirable that the resin coating layer in the second radiopaque region should differ in color from that in either of the first radiopaque region and the third radiopaque region.

It is desirable that that part of the wire proper where the radiopaque part is arranged should be at least partly tapered in such a way that the outside diameter gradually decreases in going toward the distal end.

It is desirable that the radiopaque part should be in close contact with the wire proper.
Fig. 1 is a longitudinal sectional view showing the guide wire according to the first embodiment of the present invention;
Fig. 2 is a longitudinal sectional view showing the guide wire according to the second embodiment of the present invention;
Fig. 3 is a longitudinal sectional view showing the guide wire according to the third embodiment of the present invention;
Fig. 4 is a longitudinal sectional view showing the guide wire according to the fourth embodiment of the present invention;
Fig. 5 is a longitudinal sectional view showing the guide wire according to the fifth embodiment of the present invention;
Fig. 6 is a longitudinal sectional view showing the guide wire according to the sixth embodiment of the present invention.

The guide wire according to the present invention will be described in more detail with reference to the accompanying drawings.

### <The first embodiment>

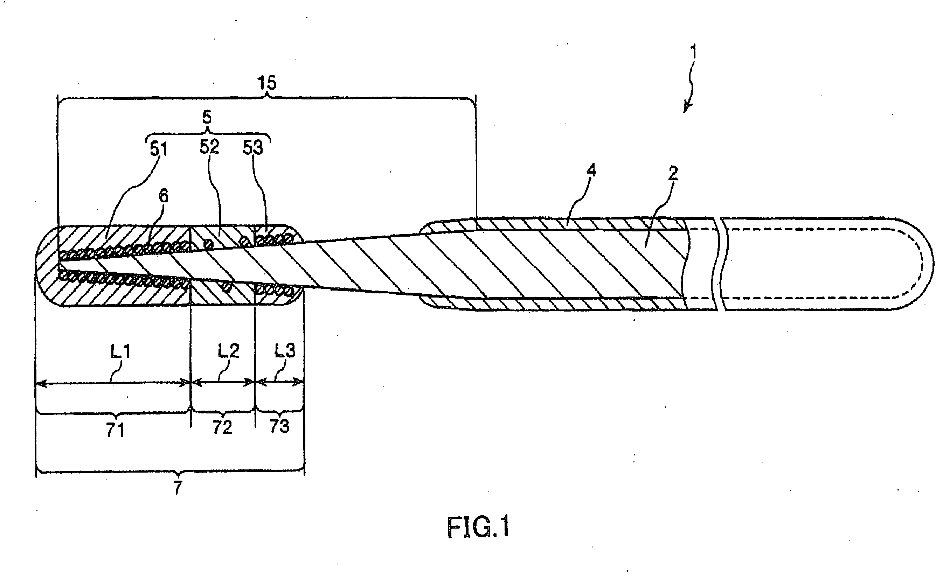

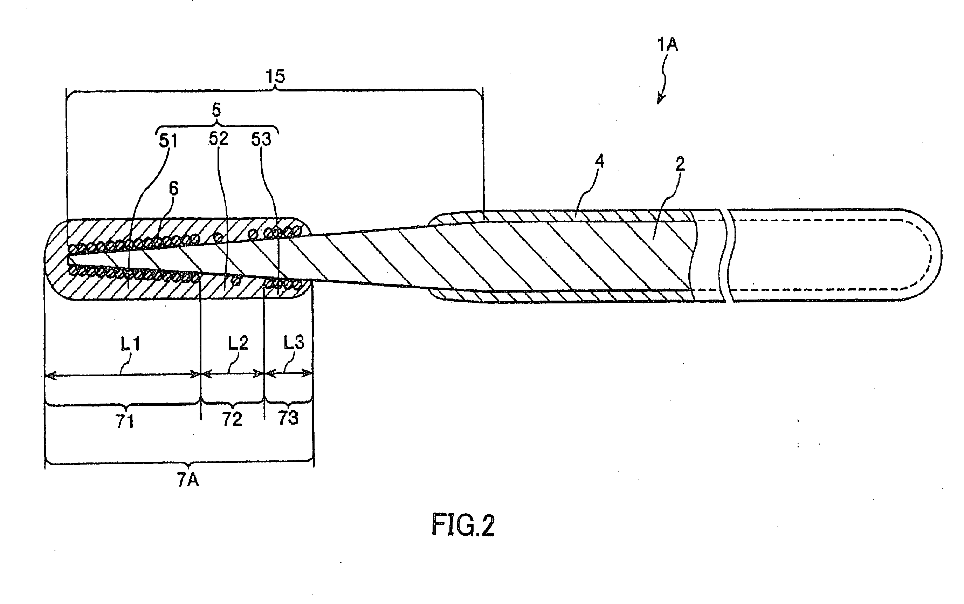

Fig. 1 is a longitudinal sectional view showing the guide wire according to the first embodiment of the present invention. For convenience' sake of explanation, the right side and the left side in Fig. 1 are called "proximal portion" and "distal end," respectively. This also applies to Figs. 2 to 6. For easy understanding, in Fig. 1 (this also applies to Figs. 2 to 6), the guide wire is schematically shown, with its length shortened and its thickness exaggerated. Therefore, the ratio of thickness to length differs from the actual one.

The guide wire 1 shown in Fig. 1 is a catheter guide wire or a transendoscopic guide wire which, at the time of use, is inserted into the lumen of a catheter (or an endoscope). It has the wire proper 2 which consists of a flexible or soft core wire (or an extended linear core). The wire proper 2 has a round shape in cross section.

According to this embodiment, the wire proper 2 consists of one continuous core wire. However, the present invention also covers the wire proper 2 consisting of two or more core wires (of identical or different materials) welded together.

The guide wire 1 should preferably have an overall length of about 200 to 5000 mm, which is not specifically restricted.

According to this embodiment, the wire proper 2 consists of two parts, the first one having a uniform outside diameter and the second one gradually tapering off toward the distal end. The second part may taper off in more than one step. The wire proper 2 shown in Fig. 1 has at its distal end one tapering part 15.

The tapering part 15 gradually decreases the flexural and torsional stiffness of the wire proper (core) 2 in going toward the distal end. This makes the guide wire 1 flexible at its distal end, which improves safety, permits the guide wire to track the blood vessel easily, and prevents the guide wire from bending. Incidentally, this tapering part 15 is where the radiopaque part 7 is arranged (mentioned later).

The illustrated guide wire is so constructed as to have the tapering part 15 at the distal end (a portion in the lengthwise direction) of the wire proper 2; however, the tapering part 15 may extend over the entire length of the wire proper 2. The tapering part 15 may decrease in outside diameter evenly or unevenly in the lengthwise direction. In other words, the angle of taper may be constant or variable from one position to another. In the latter case, steep taper and mild taper may repeat themselves more than once.

The wire proper 2 has a uniform outside diameter along its length from the proximal point of the tapering part 15 to the proximal portion.

The core wire of the wire proper 2 may be formed from any material selected from the following without specific restrictions. Stainless steel (such as SUS304, SUS303, SUS302, SUS316, SUS316L, SUS316J1, SUS316J1L, SUS405, SUS430, SUS434, SUS444, SUS429, SUS430F, and SUS302), piano wire, iron-cobalt alloy, carbon steel (including low-carbon and ultra low-carbon steels), mild steel, hard steel, ferroalloy (such as nickel steel, nickel-chromium steel, and nickel-chromium-molybdenum steel), and other alloys (such as cobalt alloy, titanium alloy, and nickel alloy). Of these examples, stainless steel is most desirable because of its higher strength and stiffness than the superelastic alloy mentioned later. It provides the guide wire 1 pushability and torque transmission performance.

The core wire of the wire proper 2 may also be formed from any alloy which exhibits pseudoelasticity, such as superelastic alloy.

Superelastic alloy is characterized by flexibility and elasticity (ability to recover itself from bending) . Thus, when applied to the wire proper 2, it makes the distal portion of the guide wire 1 sufficiently flexible and capable of restoration. The resulting guide wire 1, therefore, easily tracks the intricately winding and bending blood vessel. This leads to good steerability. In addition, because the wire proper 2 easily recovers itself from bending, the guide wire 1 is free of sustained bending that adversely affects its steerability.

The pseudoelastic alloy includes those which give any stress-strain curve due to tensile force or those which may or may not have marked transformation temperatures (such as As, Af, Ms, and Mf). It includes any alloy which greatly deforms under stress and restores its original shape almost completely when stress is removed.

Some desirable examples of the superelastic alloy are listed below. Ni-Ti alloy containing 49 to 52 at% of Ni. Cu-Zn alloy containing 38.5 to 41.5 wt% of Zn. Cu-Zn-X alloy containing 1 to 10 wt% of X (where X is at least one species selected from Be, Si, Sn, Al, and Ga). Ni-Al alloy containing 36 to 38 at% of Al. Of these examples, the Ni-Ti alloy is most desirable. Incidentally, the superelastic alloy typified by the Ni-Ti alloy is superior in adhesion to the surface layer 4 mentioned later.

Cobalt alloy has a high modulus of elasticity and an adequate elastic limit. Therefore, the wire of cobalt alloy is superior in torque transmission performance and almost free of troubles such as buckling. No restrictions are imposed on the kind of cobalt alloy so long as the alloy contains cobalt. Those alloys in which cobalt dominates (Co-based alloy: the alloy which contains cobalt dominantly by weight percent) are desirable. Co-Ni-Cr alloys are most desirable. They will enhance the above-mentioned effect. Besides, because alloys of such compositions are high in modulus of elasticity, and can be cold formed even when they are conditioned to have a high elastic limit, they permit the guide wire to be made thin while protecting it from buckling and they also impart good flexibility and stiffness necessary for the guide wire to be inserted into the desired position.

As mentioned above, the wire proper 2 may consist of two or more core wires of different materials joined together. For example, it may consist of the first core wire (extending toward the distal end) and the second core wire (extending toward the proximal portion). In this case, the first core wire should preferably be made of superelastic alloy, particularly Ni-Ti alloy, and the second core wire should preferably be made of stainless steel (mentioned above). The boundary (or junction) between the first and second core wires may exist anywhere between the tapering part 15 and the proximal portion, at the proximal point of the tapering part 15, or anywhere in the tapering part 15.

The distal end of the wire proper 2 (or the distal end of the first core wire) may have a shapeable part (not shown) which is shapeable.

The wire proper 2 may have its surface treated for good adhesion to the surface layer 4 (mentioned later). The treatment includes surface roughening treatment, chemical treatment, and heat treatment.

As shown in Fig. 1, the guide wire 1 having the wire proper 2 has the radiopaque part 7 (that produces an X-ray image) at its distal end. The radiopaque part 7 consists of the coil 6 (which is radiopaque) surrounding the wire proper 2 and the resin coating layer 5 that covers the coil 6. The radiopaque part 7 is divided into three sections the first radiopaque region 71, the second radiopaque region 72, and the third radiopaque region 73, which are arranged from the distal end in the lengthwise direction. These three regions differ in the degree of opaqueness from one another. They also differ in length from one another in the lengthwise direction of the wire proper 2.

The radiopaque part 7 has its distal end rounded to ensure the safety of the guide wire 1.

The coil 6 surrounding the distal end of the wire proper 2 is a thin wire that helically winds around the wire proper 2 in its circumferential and lengthwise directions. The coil 6 consists of a single wire that continues from the first radiopaque region 71 to the third radiopaque region 73. This structure imparts good flexibility to the radiopaque part 7, thereby contributing to safety and ability to track the lumen. This structure also reduces the number of parts constituting the radiopaque part 7 and reduces the production cost of the guide wire 1. The coil 6 may be wound tightly, loosely, and tightly in going backward from the distal end. The advantage of this structure is that the radiopaque part 7 does not abruptly change in physical properties in its lengthwise direction.

The coil 6 is made of a metallic material selected from noble metals (such as gold and platinum) and alloy thereof (such as platinum-iridium alloy) and tungsten. Thus, the coil 6 makes the guide wire 1 opaque to X-rays.

The coil 6 is formed in such a way that its inside comes into close contact with the outside of the tapering part 15 of the wire proper 2.

In this embodiment, the coil 6 is formed from a wire having a round cross section; however, the wire may have a square (or rectangular) or elliptic cross section.

The radiopaque part 7 contains the coating layer 5 that covers the coil 6. The coating layer 5 may be formed from any resin selected, without restrictions, from polyurethane, polyolefins (such as polyethylene, polypropylene, and ethylene-propylene copolymer), fluoroplastics (such as polytetrafluoroethylene), polyesters (such as polyethylene terephthalate), polyvinyl chloride, polyamide, polyimide, ethylene-vinyl acetate copolymer, ethylene-acrylonitrile-copolymer, ABS resin, AS resin, butadiene-styrene copolymer, polyisoprene, and polybutadiene. Preferable among them is a material such as polyurethane which is comparatively highly flexible because of its flexibility and superiority in adhesion to the wire 2.

The constituent of the coating layer 5 is partly incorporated with metal powder as a radiopaque radiopaque material. The metal powder may be that of tungsten or noble metal (such as gold and platinum), the former being preferable. The kind and content of the metal powder determine the opaqueness of the first to third radiopaque regions 71, 72, and 73 of the radiopaque part 7.

The radiopaque material in the coating layer 5 should have a particle size of 0.5 to 4.0 µm, preferably 1.0 to 1.5 µm, which is not specifically restricted.

The coating layer 5 may be of single-layer structure or multi-layer structure.

The wire proper 2 has its outside surface covered partly or entirely with the surface layer 4. The surface layer 4 shown in Fig. 1 covers that region of the wire proper 2 which extends from the proximal point of the tapering part 15 to the proximal portion. The surface layer 4 has multiple purposes. One major purpose is to make the guide wire 1 easily slidable with reduced friction, so that the guide wire 1 improves in steerability.

The surface layer 4 should preferably be formed from a fluoroplastic, which effectively reduces friction between the guide wire 1 and the inner wall of the catheter. This leads to improved slidability and steerability of the guide wire 1 in the catheter. In addition, reduced friction prevents the guide wire 1 from kinking and twisting which would otherwise occur (particularly near the welded part) when the guide wire 1 is moved or turned in the catheter.

The fluoroplastic includes, for example, polytetrafluoroethylene (PTFE), tetrafluoroethyleneperfluoroalkylvinyl ether copolymer (PFA), polychlorotrifluoroethylene (PCTFE), polyvinylidene fluoride (PVDF), polyvinyl fluoride (PVF), tetrafluoroethylene-hexafluoropropylene copolymer (FEP), and tetrafluoroethylene-ethylene copolymer (PETFE). They may be used alone or in combination with one another.

The guide wire 1 has its surface (at least in the region of its distal end) coated with a hydrophilic material, which gets wet to reduce the friction of the guide wire 1. This results in the guide wire 1 improving in slidability and steerability.

The hydrophilic material includes, for example, cellulosic polymer, polyethylene oxide, methylvinyl ether-maleic anhydride copolymer, polyacrylamide, polyglycidyl methacrylate-dimethylacrylamide block copolymer (PGMA-DMAA), water-soluble nylon, polyvinyl alcohol, and polyvinylpyrrolidone.

The hydrophilic materials mentioned above usually absorb water or become moistened to reduce friction between the guide wire 1 and the inner wall of the catheter or endoscope. This produces the effect of improving the slidability and steerability of the guide wire 1.

Now, as mentioned above, the radiopaque part 7 is divided into the first to third radiopaque regions 71, 72, and 73. The second one has the least opaqueness among the three. Since the first and third ones are of the same structure, the difference in opaqueness will be explained below with reference to the first one.

As shown in Fig. 1, coil 6 is wound more densely in the second radiopaque region 72 than the first radiopaque region 71, in other words, the coil 6 is tightly wound in the first radiopaque region 71 and loosely wound in the second radiopaque region 72.

Moreover, the coating layer 5 in the radiopaque part 7 varies in the content of the radiopaque material from one region to another. That is, the coating layer 52 (that portion of the coating layer 5 corresponding to the second radiopaque region 72) contains a less amount of radiopaque material than the coating layer 51 (that portion of the coating layer 5 corresponding to the first radiopaque region 71).

In this way, the radiopaque part 7 varies in opaqueness from the first radiopaque region 71 to the second radiopaque region 72 according to the winding pitch of the coil 6 and the content of the radiopaque material. Thus, the radiopaque part 7 has two regions (at its both ends), where opaqueness is high, and one region (at middle), where opaqueness is low.

The radiopaque part 7 constructed as mentioned above produces the following effect.

The radiopaque part 7 as a whole is opaque to X-rays and yet it varies in opaqueness from one region to another. The first radiopaque region 71 is more opaque, the second radiopaque region 72 is less opaque, and the third region 73 is more opaque. The difference in opaqueness produces a high contrast. The highly contrasting opaqueness permits one to distinguish the distal end (the radiopaque part 7) from the other part of the guide wire 1. Moreover, the difference in opaqueness permits one to recognize the three divided parts in the distal end of the guide wire 1. Therefore it is favorable to construct the radiopaque part 7. The second radiopaque region 72 enables one to identify (visually identify) the third radiopaque region 73 more certainly.

The radiopaque regions varying in opaqueness permit one to accurately locate the distal end and other parts of the guide wire 1 being inserted into the lumen (such as bile duct) of a living body by radioscopy, in other words, this permits one to determine how far the guide wire 1 has advanced, consequently, the guide wire can be advanced into the lumen of a living body adequately. This prevents the guide wire 1 from moving from the desired position unexpectedly.

The forgoing effect may not be produced if the radiopaque part 7 consists of the coil 6 or the radiopaque material alone. This depends on the intensity of X-rays.

According to the conventional technology, it is necessary to fix the coil 6 to the wire proper 2 with solder or adhesive, and the resultant fixing part becomes stiffer than the other parts. Moreover, the coating layers 51 and 53, which contain more radiopaque material than the coating layer 52, make the coil 6 poor in adhesion to the wire proper 2 although this depends on the constituent of radiopaque material.

In the guide wire 1 according to the present invention, however, the coating layer 72 of the radiopaque part 7 contains only a small quantity (or none) of radiopaque material and hence it firmly adheres to the coil 6 and the wire proper 2 or it helps the entire coating layer 7 to fix to the coil 6 and the wire proper 2. In addition, the coating layers 71 to 73 are bonded together by mutual dissolution of resin materials, and they (as the entire coating layer 7) firmly fix to the coil 6 and the wire proper 2. This eliminates the necessity of fixing the coil 6 with the above-mentioned fixing material, thereby preventing the distal end of the guide wire 1 from becoming stiffer. In other words, the distal end of the guide wire 1 remains flexible.

The coil 6 in the first radiopaque region 71 should be wound 3 to 7 times denser, preferably 4 to 7 times denser, that of the coil 6 in the second radiopaque region 72, although it is not specifically restricted.

The content of radiopaque material in the coating layer 52 should be less than 10%, preferably less than 5%, of that in the coating layer 51, although it is not specifically restricted. The content may be zero.

The radiopaque part 7 that produces a high contrast contributes to good steerability of the guide wire 1 being inserted into the desire position in the lumen.

As mentioned above, the first and third radiopaque regions 71 and 73 are almost identical in structure, and consequently they produce almost the same degree of contrast. This facilitates the fabrication of the radiopaque part 7.

As shown in Fig. 1, the first to third radiopaque regions 71, 72, and 73 are arranged in descending order of length. Specifically, the length (L1) of the first radiopaque region 71 is longer than the length (L2) of the second radiopaque region 72, the length (L2) of the second radiopaque region 72 is longer than the length (L3) of the third radiopaque region 73. When the guide wire 1 is inserted into the lumen of a living body, the first radiopaque region 71, which is longest, can surely be inserted and permits the guide wire 1 to be inserted deep enough to avoid slipping out. The second and third radiopaque regions 72 and 73 serve as marks to lead the guide wire 1 to the desired position. The radiopaque regions differing in length and contrast help the rapid and accurate insertion by radioscopy.

The length (L1) of the first radiopaque region 71 should be 10 to 80 mm, preferably 30 to 70 mm. The length (L2) of the second radiopaque region 72 should be 3 to 40 mm, preferably 10 to 20 mm. The length (L3) of the third radiopaque region 73 should be 1 to 20 mm, preferably 3 to 10 mm.

The ratio of L2/L1 should be 0.1 to 0.7, preferably 0.2 to 0.6. The ratio of L3/L2 should be 0.1 to 1, preferably 0.2 to 1. These ratios are necessary to produce the above-mentioned effects.

The coating layer 52 should preferably differ in color from the coating layers 51 and 53. For example, the coating layer 52 should have a chromatic color (e.g., blue) and the coating layers 51 and 53 should have an achromatic color (e.g., black). This color arrangement makes it easy to determine how far the guide wire 1 has advanced by means of an endoscope. Endoscopic observation in combination with radioscopy permits one to accurately locate the distal end of the guide wire 1.

The coloring of the coating layers 51 to 53 may be accomplished, for example, by incorporation of pigments into the resin material constituting each coating layer.

The coating layers 51 to 53, which vary in the content of radiopaque material, may be formed in the following manner which is not specifically restricted.

The wire proper 2, around which the coil 6 has been wound, is masked except for the parts where the coating layers 51 and 53 are to be formed. The resin material containing a radiopaque material is applied, except for the masked part, to form the coating layers 51 and 53.

The mask is removed, and the coating layers 51 and 53 are masked. The resin material is applied, except for the masked parts, to form the coating layer 52. In this way the coating layers 51 to 53 are formed.

According to this embodiment, the wire proper 2 is in contact with the inner surface of the coil 6. However, this is not essential. The tapering part 15 of the wire proper 2 may simply pass through the coil 6 without contact with its inner surface.

According to this embodiment, the coil 6 is formed from a single continuous wire wound around the first to third radiopaque regions 71 to 73. However, this is not essential. The coil 6 may consist of three separate coils joined together. In this case, the coils at both ends may be made of different materials.

### <The second embodiment>

Fig. 2 is a longitudinal sectional view showing the guide wire according to the second embodiment of the present invention.

The second embodiment will be described below with reference to Fig. 2. Emphasis is placed on the difference from the first embodiment mentioned above, and description of the same matter is not repeated.

The second embodiment is identical with the first one except for the structure of the radiopaque part.

In the guide wire 1A shown in Fig. 2, the radiopaque part 7A has the coating layer 5 which consists of three coating layers 51 to 53. The coating layers 51 to 53 contain substantially the same amount of the radiopaque material which is uniformly distributed therein, and there is no difference in the amount of radiopaque material in the coating layers 51 to 53. Therefore, the first and second radiopaque regions 71 and 72 differ in contrast only because the coil 6 is wound more densely in the second radiopaque region 72 than the first radiopaque region 71.

The forgoing structure is effective in the case where it is desirable in radioscopy that the radiopaque part 7A should have a lower contrast than the radiopaque part 7 in the first embodiment.

### <The third embodiment>

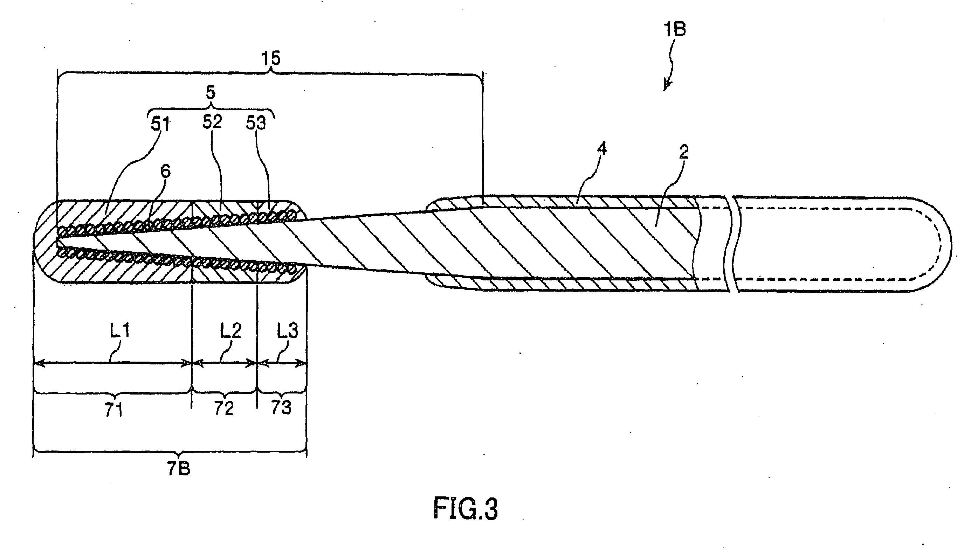

Fig. 3 is a longitudinal sectional view showing the guide wire according to the third embodiment of the present invention.

The third embodiment will be described below with reference to Fig. 3. Emphasis is placed on the difference from the first embodiment mentioned above, and description of the same matter is not repeated.

The third embodiment is identical with the first one except for the structure of the radiopaque part.

In the guide wire 1B shown in Fig. 3, the radiopaque part 7B has the coil 6 which is wound substantially uniformly (without pitch variation) throughout the first to third radiopaque regions 71 to 73. Thus, in the radiopaque part 7B, difference in opaqueness between the first radiopaque region 71 and the second radiopaque region 72 is due mainly to the fact that the coating layer 52 contains a less amount of radiopaque material than the coating layer 51.

The forgoing structure is effective in the case where it is desirable in radioscopy that the radiopaque part 7B should have a lower contrast than the radiopaque part 7 in the first embodiment.

### <The fourth embodiment>

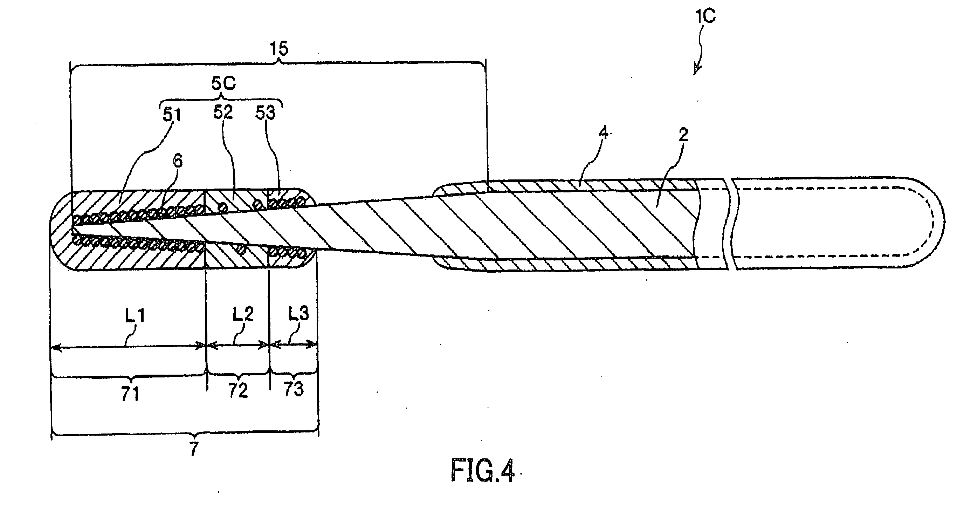

Fig. 4 is a longitudinal sectional view showing the guide wire according to the fourth embodiment of the present invention.

The fourth embodiment will be described below with reference to Fig. 4. Emphasis is placed on the difference from the first embodiment mentioned above, and description of the same matter is not repeated.

The fourth embodiment is identical with the first one except for the structure of the resin coating layer.

In the guide wire 1C shown in Fig. 4, the coating layers 51 to 53 contain almost the same amount of radiopaque material. However, the radiopaque material in the coating layer 52 is poorer in opaqueness than that in the coating layers 51 and 53. The difference in opaqueness and the difference in the winding pitch of the coil 6 produce a synergistic effect of distinguishing between the first radiopaque region 71 and the second radiopaque region 72. Therefore, as in the first embodiment, the radiopaque part 7 produces a good contrast in radioscopy. On account of the radiopaque part 7 which produces a good contrast and has the radiopaque regions varying in length, the guide wire 1C permits one to accurately locate it in the lumber of a living body.

The coating layers 51 and 52 can be made to vary in opaqueness by incorporating them with a radiopaque material differing in composition. For example, their radiopaque material may be tungsten and bismuth sulfide, respectively, or tungsten and barium sulfide, respectively.

### <The fifth embodiment>

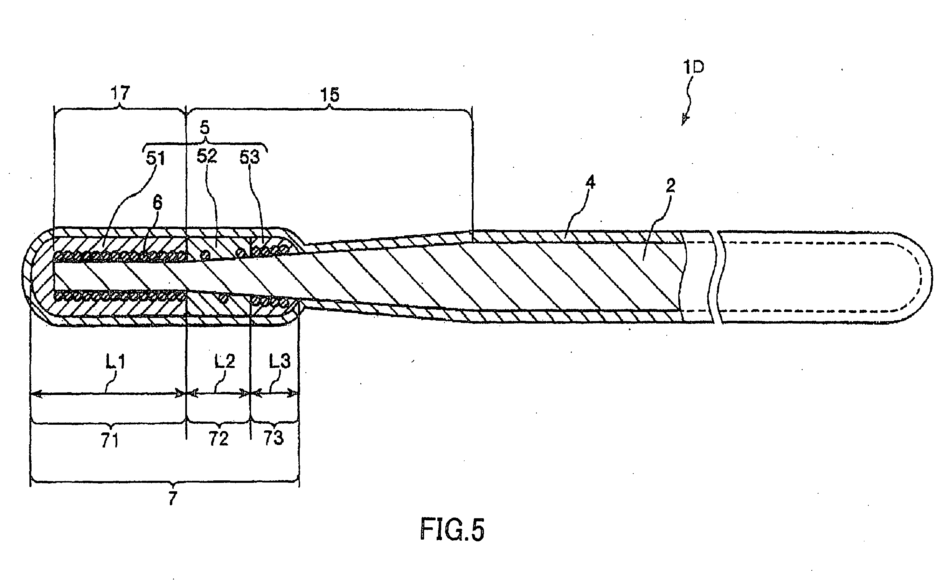

Fig. 5 is a longitudinal sectional view showing the guide wire according to the fifth embodiment of the present invention.

The fifth embodiment will be described below with reference to Fig. 5. Emphasis is placed on the difference from the first embodiment mentioned above, and description of the same matter is not repeated.

The fifth embodiment is identical with the first one except for the shape of the distal end of the wire proper.

In the guide wire 1D shown in Fig. 5, the wire proper 2 has at its distal end the part 17 which has a uniform outside diameter and extends from the end of the tapering part 15 to the distal end. This structure makes the distal end of the wire proper 2 gradually decrease in stiffness in going toward the distal end. As the result, the guide wire 1D has good flexibility at its distal end and easily tracks the blood vessel without bending, which leads to greater safety.

The radiopaque part 7 is so formed as to step over the part 17 (which has a uniform outside diameter) and a portion of the tapering part 15. It consists of the first to third radiopaque regions 71 to 73. The first radiopaque region 71 is in the part 17, and the second and third radiopaque regions 72 and 73 are in the tapering part 15.

In the case shown in Fig. 5, the part 17 is formed at the distal end of the wire proper 2; however, it may also be formed in any part of the wire proper 2 (for example, in the middle of the tapering part 15).

The surface layer 4 covers the guide wire 1D entirely (from the distal end to the proximal portion). It reduces the friction (sliding resistance) of the guide wire 1 and improves slidability, which contributes to the steerability of the guide wire 1.

### <The sixth embodiment>

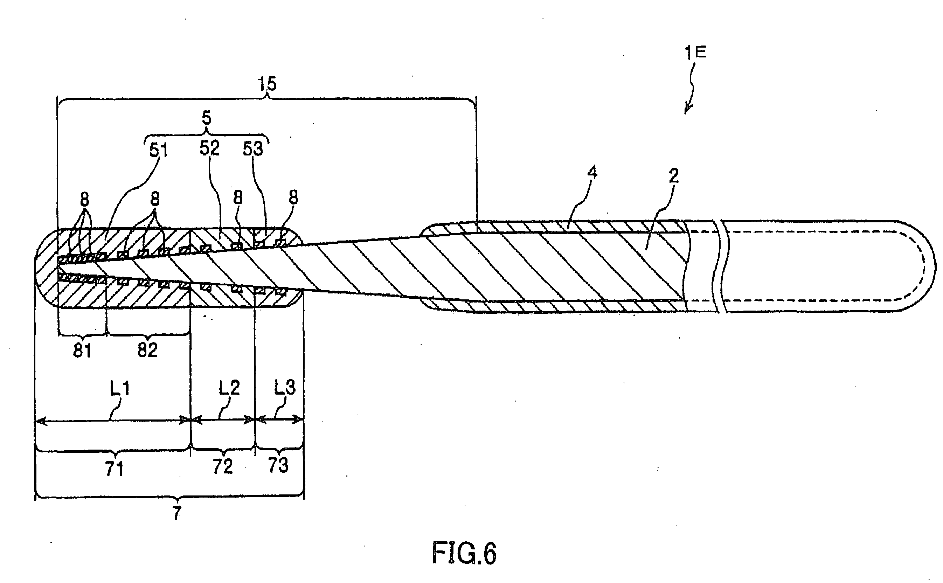

Fig. 6 is a longitudinal sectional view showing the guide wire according to the sixth embodiment of the present invention.

The sixth embodiment will be described below with reference to Fig. 6. Emphasis is placed on the difference from the first embodiment mentioned above, and description of the same matter is not repeated.

The sixth embodiment is identical with the first one except for the structure of the radiopaque member.

The guide wire 1E shown in Fig. 6 has ringlike members 8 which are opaque to X-rays. The ringlike members are arranged in the lengthwise direction of the wire proper 2 and inserted on the tapering part 15 of the wire proper 2. An inner circumference surface of ringlike members 8 is intimately contacted with an outer circumference surface of the wire proper 2.

The ringlike members 8 are arranged more closely, on average, in the second radiopaque region 72 than in the first and third radiopaque regions 71 and 73. Moreover, the first radiopaque region 71 consists of subsections 81 and 82. In the subsection 81, the adjacent ringlike members 8 are in contact with one another. In the subsection 82, the adjacent ringlike members 8 are spaced from one another. The subsection 81 is closer to the distal end than the subsection 82.

The guide wire 1E is **characterized in that** the ringlike members are arranged at intervals that vary from the first radiopaque region 71 to the second radiopaque region 72. This difference in intervals combined with the difference in the content of radiopaque material makes an apparent difference in opaqueness between the first radiopaque region 71 and the second radiopaque region 72. Thus the radiopaque part 7 produces a good contrast as in the first embodiment. Owing to the radiopaque part 7 excelling in opaqueness and the radiopaque part 7 consisting of radiopaque regions differing in length, the guide wire 1E permits one to accurately locate its position by radioscopy at the time of its insertion into the lumen of a living body.

The ringlike members 8 functioning as the radiopaque members in the guide wire 1E can be easily arranged at varying intervals.

The ringlike members 8 may be formed from any material, which includes such material as used for the coil 6 in the first embodiment.

In this embodiment, the ringlike members 8 have a square or rectangular cross section; however, the cross section may be round or elliptic.

In the foregoing, the guide wire according to the present invention has been described with reference to the illustrated embodiments. The present invention is not limited to them. The constituents of the guide wire may be replaced by those which have the same functions or may be modified with additional elements.

The present invention may have any two or more structures (features) in combination selected from the foregoing embodiments.

The guide wire according to the present invention is not limited to the one which is used for transendoscopic technique. It can also be used for therapy of CTO (chromic total occlusion) and angiography and PTCA (Percutaneous Transluminal Coronary Angioplasty).

According to the foregoing embodiments, the radiopaque part has three radiopaque regions. However, it may have one or two additional ones next to the third one. In this case, the adjacent radiopaque regions should preferably differ in opaqueness from one another.

According to the foregoing embodiments, the second radiopaque region is longer than the third one. However, both may have the same length.

According to the foregoing embodiments, the radiopaque part has the radiopaque members and the coating layer. However, either of them may be omitted.

According to the foregoing embodiments, the first and third radiopaque regions have approximately the same degree of opaqueness. However, the first region may have a higher or lower degree of opaqueness than the third one.

According to the foregoing embodiments, the coating layer contains metal powder as a radiopaque material. However, the metal powder may be replaced by any metal oxide power opaque to X-rays. The metal oxide powder includes, for example, barium sulfide, bismuth oxide, and barium carbonate. Of those, the barium sulfide and the bismuth oxide are preferable. More than one kind of radiopaque material may be used in combination. For example, the coating layer in the first and third radiopaque regions may contain tungsten power (metal powder) as a radiopaque material and the coating layer in the second radiopaque region may contain barium sulfate powder (metal oxide powder) as a radiopaque material.

It should be understood by those skilled in the art that various modifications, combinations, subcombinations and alterations may occur depending on design requirements and other factor in so far as they are within the scope of the appended claims .

## Claims

1. A guide wire (1) having a long wire proper (2) and also having a radiopaque part (7) capable of forming an X-ray image formed at the distal end thereof,
said radiopaque part (7) having at least one radiopaque member composed of a metallic material capable of forming an X-ray image, which is arranged outside said wire proper (2) and in the lengthwise direction of said wire proper (2), and also having a resin coating layer (5) which covers said at least one radiopaque member (7) and at least part of which contains a radiopaque material capable of forming an X-ray image, and said radiopaque part (7) being divided into a first radiopaque region (71), a second radiopaque region (72), and a third radiopaque region (73) which are sequentially arranged from the distal end in the lengthwise direction of said wire proper (2),
said second radiopaque region (72) being less capable of forming an X-ray image than the first radiopaque region (71) and the third radiopaque region (73),
**characterized in that**
the difference in capability of forming an X-ray image between the first radiopaque region (71) and the second radiopaque region (72) is due to the fact that said at least one radiopaque member in said second radiopaque region (72) is arranged less densely than that in said first radiopaque region (71) and that either said resin coating layer (5) in said second radiopaque region (72) contains a lesser amount of radiopaque material than said resin coating layer (5) in said first radiopaque region (71), or that said radiopaque material in said resin coating layer (5) in said second radiopaque region (72) is less capable of forming an X-ray image than said radiopaque material in said resin coating layer (5) in said first radiopaque region (71).

2. The guide wire (1) as defined in Claim 1, wherein said radiopaque material is particles of a metallic material or a metal oxide.

3. The guide wire (1) as defined in any of Claims 1 to 2, wherein said radiopaque member (8) is a helically wound coil (6) which is wound less densely in said second radiopaque region (72) than said first radiopaque region (71).

4. The guide wire (1) as defined in Claim 3, wherein said coil (6) is formed from a single filamentous body that extends from said first radiopaque region (71) to said third radiopaque region (73).

5. The guide wire (1) as defined in any of Claims 1 to 2, wherein said radiopaque member (8) takes on a ringlike shape and a plurality of said radiopaque members are arranged in the lengthwise direction of said wire proper (2), and adjacent radiopaque members (8) are arranged less densely in said second radiopaque region (72) than in said first radiopaque region (71).

## Patentansprüche

1. Führungsdraht (1), der einen langen Drahtkörper (2) aufweist und der auch ein strahlenundurchlässiges Stück (7) aufweist, das zum Ausbilden eines Röntgenbildes fähig ist, das an seinem Distalende ausgebildet wird,
wobei das strahlenundurchlässige Stück (7) mindestens ein strahlenundurchlässiges Bauteil aufweist, das aus einem metallischen, zum Ausbilden eines Röntgenbildes fähigen Material aufgebaut ist, welches außerhalb des Drahtkörpers (2) und in der Längsrichtung des Drahtkörpers (2) angeordnet ist und welches auch eine Gummideckschicht (5) aufweist, die das mindestens eine strahlenundurchlässige Bauteil (7) abdeckt und von der mindestens ein Stück ein strahlenundurchlässiges Material enthält, das zum Ausbilden eines Röntgenbildes fähig ist, und wobei das strahlenundurchlässige Stück (7) in einen ersten strahlenundurchlässigen Bereich (71), einen zweiten strahlenundurchlässigen Bereich (72) und einen dritten strahlenundurchlässigen Bereich (73) unterteilt ist, die aufeinander folgend von dem Distalende aus in der Längsrichtung des Drahtkörpers (2) angeordnet sind,
wobei der zweite strahlenundurchlässige Bereich (72) weniger zum Ausbilden eines Röntgenbildes fähig ist als der erste strahlenundurchlässige Bereich (71) und der dritte strahlenundurchlässigen Bereich (73),
**dadurch gekennzeichnet, dass**
der Unterschied in der Fähigkeit zum Ausbilden eines Röntgenbildes zwischen dem ersten strahlenundurchlässigen Bereich (71) und dem zweiten strahlenundurchlässigen Bereich (72) darauf zurückzuführen ist, dass das mindestens eine strahlenundurchlässige Bauteil in dem zweiten strahlenundurchlässigen Bereich (72) weniger dicht angeordnet ist als jenes in dem ersten strahlenundurchlässigen Bereich (71) und dass entweder die Gummideckschicht (5) in dem zweiten strahlenundurchlässigen Bereich (72) eine geringere Menge an strahlenundurchlässigem Material enthält als die Gummideckschicht (5) in dem ersten strahlenundurchlässigen Bereich (71) oder das strahlenundurchlässige Material in der Gummideckschicht (5) in dem zweiten strahlenundurchlässigen Bereich (72) weniger zum Ausbilden eines Röntgenbildes fähig ist als das strahlenundurchlässige Material in der Gummideckschicht (5) in dem ersten strahlenundurchlässigen Bereich (71).

2. Führungsdraht (1) nach Anspruch 1, wobei das strahlenundurchlässige Material Teilchen eines metallischen Materials oder eines Metalloxids sind.

3. Führungsdraht (1) nach einem der Ansprüche 1 bis 2,
wobei das strahlenundurchlässige Bauteil (8) eine wendelförmig gewickelte Spule (6) ist, die in dem zweiten strahlenundurchlässigen Bereich (72) weniger dicht gewickelt ist als in dem ersten strahlenundurchlässigen Bereich (71).

4. Führungsdraht (1) nach Anspruch 3, wobei die Spule (6) aus einem einzigen faserigen Körper gebildet ist, der sich vom ersten strahlenundurchlässigen Bereich (71) zum dritten strahlenundurchlässigen Bereich (73) erstreckt.

5. Führungsdraht (1) nach einem der Ansprüche 1 bis 2,
wobei das strahlenundurchlässige Bauteil (8) eine ringförmige Gestalt annimmt und mehrere von den strahlenundurchlässigen Bauteilen in der Längsrichtung des Drahtkörpers (2) angeordnet sind und wobei die benachbarten strahlenundurchlässigen Bauteile (8) in dem zweiten strahlenundurchlässigen Bereich (72) weniger dicht angeordnet sind als in dem ersten strahlenundurchlässigen Bereich (71).

## Revendications

1. Fil de guidage (1) comportant un fil long (2) et comportant aussi une partie radio-opaque (7) capable de former une image radiographique formée à son extrémité distale,
ladite partie radio-opaque (7) comportant au moins un élément radio-opaque composé d'un matériau métallique capable de former une image radiographique, qui est placé à l'extérieur dudit fil (2) et dans la direction de la longueur dudit fil (2), et comportant en outre une couche de revêtement en résine (5) qui couvre ledit au moins un élément radio-opaque (7) et dont au moins une partie contient un matériau radio-opaque capable de former une image radiographique, et ladite partie radio-opaque (7) étant divisée en une première région radio-opaque (71), une deuxième région radio-opaque (72) et une troisième région radio-opaque (73) qui sont disposées les unes à la suite des autres depuis l'extrémité distale dans la direction de la longueur dudit fil (2),
ladite deuxième région radio-opaque (72) étant moins capable de former une image radiographique que la première région radio-opaque (71) et la troisième région radio-opaque (73),
**caractérisé en ce que :**
la différence de capacité à former une image radiographique entre la première région radio-opaque (71) et la deuxième région radio-opaque (72) est due au fait que ledit au moins un élément radio-opaque de ladite deuxième région radio-opaque (72) est agencé de façon moins dense que celui de ladite première région radio-opaque (71) et que soit ladite couche de revêtement en résine (5) dans ladite deuxième région radio-opaque (72) contient une quantité moindre de matériau radio-opaque que ladite couche de revêtement en résine (5) dans ladite première région radio-opaque (71), soit ledit matériau radio-opaque de ladite couche de revêtement en résine (5) dans ladite deuxième région radio-opaque (72) est moins capable de former une image radiographique que ledit matériau radio-opaque de ladite couche de revêtement en résine (5) dans ladite première région radio-opaque (71).

2. Fil de guidage (1) selon la revendication 1, dans lequel ledit matériau radio-opaque est constitué de particules d'un matériau métallique ou d'un oxyde métallique.

3. Fil de guidage (1) selon la revendication 1 ou 2, dans lequel ledit élément radio-opaque (8) est une bobine enroulée de façon hélicoïdale (6) qui est enroulée de manière moins dense dans ladite deuxième région radio-opaque (72) que dans ladite première région radio-opaque (71).

4. Fil de guidage (1) selon la revendication 3, dans lequel ladite bobine (6) est formée à partir d'un seul corps filamenteux qui s'étend de la première région radio-opaque (71) à la troisième région radio-opaque (73).

5. Fil de guidage (1) selon la revendication 1 ou 2, dans lequel ledit élément radio-opaque (8) prend une forme de bague et une pluralité desdits éléments radio-opaques sont disposés dans la direction de la longueur dudit fil (2), et les éléments radio-opaques (8) adjacents sont disposés de façon moins dense dans ladite deuxième région radio-opaque (72) que dans ladite première région radio-opaque (71).
